# EUROPEAN PATENT SPECIFICATION

(11) **EP 3 459 495 B1**
(45) Date of publication and mention of the grant of the patent: **18.05.2022**
(21) Application number: 18195291.2
(22) Date of filing: 18.09.2018
(51) Int. Cl.: A61F 2/00

(54) **MESH SURGICAL DEVICE FOR LAPAROSCOPIC USE**
CHIRURGISCHE NETZVORRICHTUNG FÜR LAPAROSKOPISCHE ANWENDUNG
DISPOSITIF CHIRURGICAL TRICOTÉ POUR USAGE LAPAROSCOPIQUE

(30) Priority: 20.09.2017 IT 201700105278
(43) Date of publication of application: 27.03.2019
(73) Proprietor: Tola, Nardo, 09045 Quartu Sant'Elena (CA) (IT)
(72) Inventor: Tola, Nardo, 09045 Quartu Sant'Elena (CA) (IT)
(74) Representative: Crugnola, Pietro

(56) References cited:
- EP-A1- 2 517 670
- WO-A1-03/068107
- WO-A1-2006/045042
- WO-A2-2007/149348
- WO-A2-2008/058163
- FR-A1- 2 852 818
- US-A1- 2008 140 218

## Description

The invention relates to a mesh surgical device for laparoscopic use, in particular a mesh surgical device usable for correcting laparoscopically a pelvic organ prolapse, and more in particular a mesh surgical device usable for correcting laparoscopically a multicompartment pelvic organ prolapse.

The pelvic organ prolapse (POP) is a pathological condition that can be found frequently in the female sex, in which one or more pelvic organs move irreversibly from their physiological position and protrude into the vaginal lumen. This occurs when the muscles, the ligaments and the fascia (pubocervical fascia) that support the aforesaid organs in correct position undergo to failure and weaken. The aforesaid prolapse can affect the uterus (hysterocele), the bladder (cystocele), the small intestine (enterocele) and the rectum (rectocele). The pelvic organ prolapse is defined as "multicompartment" when it involves a plurality of pelvic districts, and consequently a plurality of pelvic organs, causing a plurality of symptoms: urinary incontinence, difficulty in urinating, chronic constipation, obstructed defecation syndrome (ODS).

For the treatment of the pelvic organ prolapse transvaginal surgery techniques have been devised and used, namely operating techniques that are carried out through the vaginal cavity. The known transvaginal surgery techniques comprise the transvaginal fascia surgery and the prosthetic transvaginal surgery. In the transvaginal fascia surgery, the supporting tissue structures of the prolapsed organs are reconstructed without the use of prostheses (for example: fascia duplication according to Kelly, unilateral sacrospinous fixation, bilateral sacrospinous fixation, etc). The aforesaid fascia surgery has significant drawbacks, such as: major traumatism, high rate of relapses, substantially long hospitalization (7-10 days), urine retention and postoperative pain.

In order to try to overcome the drawbacks connected with the transvaginal fascia surgery, techniques of prosthetic transvaginal surgery have been subsequently proposed, namely operating techniques based on the use of implantable prostheses. The latter consist of meshes of synthetic material (biocompatible polymers), that are shaped (cut) directly by the surgeon at the moment of use and interposed in the vescicovaginal or rectovaginal space, so as to create a fascia reinforcement. In order to improve the practicality of use of the aforesaid meshes, pre-shaped, namely pre-cut, meshes have been made.

The use of an implantable prosthesis, made in the form of a mesh, has enabled better results to be obtained than those obtainable through the transvaginal fascia surgery. Nevertheless, also the prosthetic transvaginal surgery resulted to be affected by drawbacks, such as non-positive outcomes of the intervention and not negligible frequency of complications. In particular, in July 2011 the United States FDA (Food and Drug Administration), after detecting many cases of postoperative complications, expressed considerable concern about the risks associated with the prosthetic transvaginal surgical treatment of the pelvic organ prolapse.

In order to overcome the drawbacks disclosed above, prosthetic surgical techniques have been proposed that are alternative to the transvaginal one. In particular, the surgical technique known as "POPS" (Pelvic Organ Prolapse Suspension), devised by Dr Antonio Longo, and the surgical technique known as "LLS" (Laparoscopic Lateral Suspension), devised by Dr J.B. Dubuisson, are known. The two techniques are similar and provide for an intervention in laparoscopy, during which a mesh of biocompatible material is positioned below the peritoneal membrane, so as to replace and reinforce the broken ligaments. Both the techniques aim to bring the (prolapsed) uterus back to the anatomical seat thereof, consequently bringing also the bladder and the rectum back to the correct position.

The POPS technique provides for the use of an angular simple two arm-mesh made of polypropylene, which is 2 cm wide and cut at the moment of use from a square mesh (dimensions: 30 x 30 cm). Pre-shaped (namely pre-cut) embodiments of this mesh are also known. Similarly to the POPS technique, also the LLS technique provides for the use of a simple two-arm mesh.

Although the POPS and LLS surgical techniques turned out more effective than the prosthetic transvaginal surgery, nevertheless the meshes made of biocompatible material currently used in these laparoscopic techniques have significant drawbacks.

From the surgical experience of the Applicant, it has been found that the mesh used in the POPS technique is not able to appropriately correct a condition of advanced cystocele, especially if the aforesaid condition is associated with the structural failure of the pubocervical fascia and the detachment of the vaginal wall from the tendinous arch. More in general, the Applicant believes that the use of the meshes of known type is not sufficient in the treatment of a high-degree multicompartment pelvic organ prolapse, especially if the prolapse is associated with an important lateral and/or anterior defect, urinary incontinence and ODS.

US 2008/140218 A1 discloses an implant for treating central and lateral cystoceles present in a female patient, comprising a body having a pair of opposed ends, one of said ends having a concave shape selected so as to form an unobstructed recess which avoids contact with the patient's bladder neck, a central longitudinal axis passing through said pair of opposed ends, an inboard area located between said opposed ends, said inboard area containing said longitudinal axis and having a size and shape selected so as to repair a central cystocele, at least one outboard area positioned on at least one side of said inboard area, said at least one outboard area having a size and shape selected so as to repair a lateral cystocele, and stabilizing means for laterally stabilizing said body on both sides thereof independently of the patient's arcus tendineous fascia pelvis.

WO 03/068107 A1 discloses a surgical kit including a mesh, which is usable for restoring a prolapsed organ within a patient's pelvic cavity. The mesh includes a support sheet portion to be positioned beneath the organ, first and second front attachment strips extending from a proximal region of the mesh and first and second rear attachment strips extending form a distal region of the mesh.

WO 2007/149348 A2 discloses a multi-piece pelvic implant comprising a tissue support portion and an extension portion, the implant comprising pieces that include: a support portion piece comprising a tissue support portion and optional support portion piece arm, and an extension portion piece, wherein the extension portion piece is adjustably connected to the support portion piece, and includes a frictional adjusting element that allows adjustment of a length of the extension portion, the frictional adjusting element comprising an aperture through which a segment of extension portion extends and a surface that frictionally engages the segment of extension portion, wherein the frictional engagement preferentially allows movement of the segment of extension portion through the aperture in one direction and inhibits movement of the segment of extension portion in an opposing direction.

FR 2 852 818 A1 discloses an implant for treating cystocele, having two anterior sling attachments and two posterior sling attachments disposed in sagittal plane, where each attachment extends from a side of a support body. Two sling attachment units extend from the body and are disposed in the sagittal plane between the sling attachments. A longitudinal axis of the unit forms a predetermined angle with an anterior part of the plane. WO 2006/045042 A1 discloses an implantable graft for treatment of pelvic organ prolapse, comprising: a body portion, comprising a planar section having a longitudinal axis, opposed first and second ends, and opposed first and second lateral edges that terminate at said first and second ends; and opposed first and second apical arms, wherein said first apical arm extends from said first lateral edge at a first interface closer to said first end of said body portion than said second end, wherein said second apical arm extends from said second lateral edge at a second interface closer to said first end of said body portion than said second end.

EP 2 517 670 A1 discloses a prosthesis, in particular for urogynecological and andrological treatments, comprising: an intermediate portion having, in use, a supporting function for a part of the body; at least two lateral arms that project in a cantilever fashion from the intermediate portion and have respective external end portions defining connecting areas and made in the guise of a mesh of non-resorbable biocompatible polymer material. The intermediate portion comprises a film made of non-resorbable biocompatible polymer material fixed to the mesh.

WO 2008/058163 A2 discloses an implant for treating a pelvic floor disorder in a patient comprising: a body portion; a first pair of arms extending from said body portion; a second pair of arms extending from said body portion; a third pair of arms extending from said body portion. The body portion is more flexible than said first, second and third pair of arms.

From what has been set forth above, there is thus a need to overcome the significant drawbacks connected with known mesh devices for the treatment of the pelvic organ prolapse in patients of female sex. More exactly, the implantable mesh devices used until now for the laparoscopic surgical treatment of the pelvic organ prolapse turn out substantially not sufficient for achieving really satisfactory and durable results, in particular in the cases of prolapse of 3^{rd}-4^{th} degree with major urinary incontinence or ODS and with a significant increase in endoabdominal pressure, which occurs especially in overweight patients.

An object of the invention is to improve the mesh surgical devices that are usable for the treatment of the pelvic organ prolapse.

Another object is to improve the mesh surgical devices that are usable for the laparoscopic treatment of the pelvic organ prolapse.

A further object is to make available a mesh surgical device for laparoscopic use that is effectively usable for the treatment of the high-degree multicompartment pelvic organ prolapse.

According to the invention, a mesh surgical device is provided, as defined in claim 1. Owing to the invention, a mesh surgical device for laparoscopic use is made available, through which it is possible to overcome the drawbacks of the prior art.

The Applicant, based on the surgical experience thereof, namely on the performance of over 300 interventions of laparoscopic suspension for the correction of various types of pelvic organ prolapse, concluded that the known mesh devices (two-arm meshes) are not suitable for treatment of all the types and degrees of prolapse. On the contrary, to obtain an optimum result, it is necessary to use various types of mesh, having configurations that come closer to the peculiarity, as well as the degree, of the anatomical defect to be corrected.

In particular, by taking account of the anatomy and physiology of the damaged ligaments of the pelvic floor (on which type and degree of prolapse depend), the Applicant has devised a mesh surgical device for laparoscopic use, comprising a main or central part, intended to be fixed (by surgical suture) to the pubocervical fascia and to the uterine isthmus (or to the vaginal dome in hysterectomized patients), and a variable number of side arms. In particular, the side arms are configured in pairs. The pairs of side arms enable the deficit to be remedied that is caused by the damaged ligaments and to correct simultaneously many of the defects that are present in the multicompartment pelvic organ prolapse. The side arms lead from opposite sides of the central body and the (variable) number of the aforesaid side arms is a number equal to two, four or six. The mesh surgical device for laparoscopic use according to the invention is provided with a number of pairs of side arms equal to three.

The invention can be better understood and implemented with reference to the attached drawings that illustrate an embodiment thereof by way of non-limiting example, in which:
Figure 1 is a schematic plan view of an embodiment of the mesh surgical device that does not form part of the invention, which is usable to correct a condition of central defect;
Figure 2 is a schematic plan view of another embodiment of the mesh surgical device that does not form part of the invention, which is usable to correct a condition of anterior defect;
Figure 3 is a schematic plan view of a further embodiment of the mesh surgical device that does not form part of the invention, which is usable to correct a condition of lateral defect;
Figure 4 is a schematic plan view of an embodiment of the mesh surgical device according to the invention, which is usable to correct a condition of total defect.

In the context of the present description and the attached claims, the terms "mesh surgical device for laparoscopic use" and "mesh device" are considered to be synonyms and are thus used in a mutually interchangeable manner.

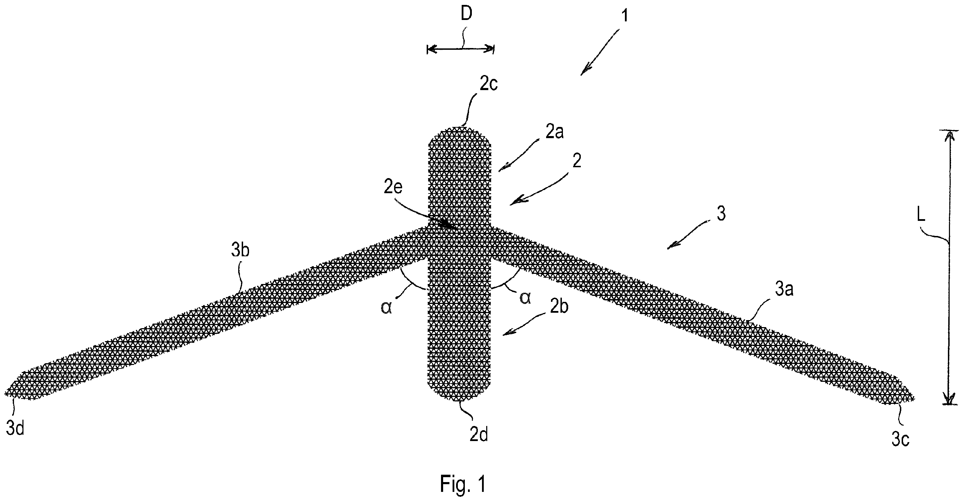

Figure 1 shows a mesh surgical device for laparoscopic use (mesh device) 1 (that does not form part of the invention), made of a suitable apyrogenic and atoxic synthetic polymer, for example non-reabsorbable monofilament polypropylene, sterilized through known procedures. The mesh device 1 comprises a portion or central body 2 and a first pair of appendages or side arms 3.

The central body 2 is approximately quadrilateral-shaped and comprises a first portion 2a and a second portion 2b, which are reciprocally aligned and opposite. The central body 2 comprises a first free end 2c and a second free end 2d, which are respectively comprised in the first portion 2a and in the second portion 2b. The first free end 2c and the second free end 2d are convex, in particular rounded. In embodiments that are not shown, the first free end 2c and/or the second free end 2d are not convex or rounded but can be, for example, rectilinear or concave.

Between the first portion 2a and the second portion 2b a connecting portion 2e is interposed, which is approximately quadrilateral-shaped and from opposite sides of which the first pair of side arms 3 leads. The first portion 2a, the second portion 2b and the connecting portion 2e have a same transverse dimension or width D (indicated by a double-tipped arrow, only in Figure 1), which is the transverse dimension (or width) D of the central body 2. Moreover, the first portion 2a, the second portion 2b and the connecting portion 2e (that are reciprocally aligned) define altogether a longitudinal dimension or length L (indicated by a further double-tipped arrow, only in Figure 1) of the central body 2. In the embodiments of the mesh device 1 shown in Figures 1-4, the transverse dimension D is 35 mm, whereas the longitudinal dimension L is 152 mm (15.2 cm). In embodiments that are not shown, in the central body of the mesh device according to the invention the transverse dimension and/or the longitudinal dimension can be greater or less than those disclosed above. For example, the transverse dimension or width of the first portion, of the connecting portion and/or of the second portion can be different.

The first pair of side arms 3 comprises a first side arm 3a and a second side arm 3b, each of which is quadrilateral-shaped, particularly extended in length and approximately ribbon shaped. In the embodiments of the mesh device shown in Figures 1-4, each side arm 3a, 3b has a transverse dimension or width of 17 mm and a longitudinal dimension or length of 250 mm (25 cm). In embodiments that are not shown, the width and/or the length can be greater or less than those disclosed above and, for example, the two side arms of the first pair can have different widths and/or lengths from one another.

Each side arm 3a, 3b comprises a free end 3c, 3d with an acute angle and an opposite end that is joined to a corresponding side of the connecting portion 2e. Therefore, the side arms 3a, 3b lead from opposite sides of the connecting portion 2e and, consequently, from opposite sides of the central body 2. In embodiments that are not shown, each side arm has a free end that is not with an acute angle but can be, for example, rectilinear, convex or concave.

Each side arm 3a, 3b leads obliquely from the corresponding side of the central body 2, in such a manner that a first angle α less than 90°, and in particular a first angle α of about 70°, is defined between each side arm 3a, 3b and the central body 2. More exactly, the aforesaid first angle α (of about 70°) is defined between each side arm 3a, 3b and the second portion 2b. Consequently, as it is visible in Figures 1-4, each side arm 3a, 3b leads obliquely from the corresponding side of the central body 2 in such a manner that the corresponding free end 3c, 3d is positioned at about the level of the second free end 2d of the central body 2. In embodiments that are not shown, the first angle defined between each side arm and the central body is less than 90° and greater or less than 70°. In other embodiments that are not shown, the first angle defined between each side arm and the central body can be 90°.

It should be noted that, in the embodiments of the mesh device shown in Figures 1-4, the first portion 2a and the second portion 2b have a different length. In particular the first portion 2a is shorter than the second portion 2b (55 mm versus 80 mm). This means that, in the embodiments shown in Figures 1-4, the connecting portion 2e is not in an exactly intermediate position between the first portion 2a and the second portion 2b, namely in an intermediate position between the first free end 2c and the second free end 2d of the central body 2, but it is in a position nearer the first free end 2c (as shown, for example, in Figure 1). Consequently, each side arm 3a, 3b leads obliquely from a zone of the central body 2 nearer the first free end 2c. The greater length of the second portion 2b enables the latter to be used effectively in all the cases in which it is necessary to reinforce the rear vaginal wall and, in particular, in the event of a prolapse of the vaginal dome in hysterectomized women. In embodiments that are not shown, the first portion and the second portion of the central body can be of the same length, or the first portion can be longer than the second portion.

In use, namely during the surgical intervention in laparoscopy, the mesh device 1 can be oriented by the surgeon in such a manner that the first portion 2a is "anterior", namely facing the anterior abdominal wall of a patient (into the body cavity of which the mesh device according to the invention is implanted), and the second portion 2b is "posterior", i.e. facing the posterior abdominal wall of the patient.

The mesh device 1 disclosed above is usable in cases of POP involving young women of childbearing age and with 1^{st}-2^{nd} degree hysterocele and cystocele with a prevalent central defect. They are patients who have had one or more births, the births often being dystocial and with macrosomic foetuses, and who complain of a sense of genital heaviness, dyspareunia, pollakiuria, nycturia, 1^{st} degree occasional or frequent SUI (stress urinary incontinence).

In laparoscopy, by (reabsorbable or non-reabsorbable) plurifilament suture points, the anterior part of the central body 2, i.e. the first portion 2a, is fixed to the pubocervical fascia, subject to disconnection of the latter from the vesical wall, and the connecting portion 2e of the central body 2 is fixed centrally to the uterine isthmus (if present) or to the apex of the vaginal dome (in hysterectomized patients). The aforesaid disconnection is performed proportionally to the size of the cystocele. The first portion 2a of the central body 2 exerts a reinforcing action on the anterior vaginal wall. Before use, if the size of the fascia to be reinforced is less than the length of the first portion 2a, the dimensions of the latter can be reduced (i.e. cut out) directly by the surgeon.

The rear part of the central body 2, i.e. the second portion 2b, is removed, if the uterus is present, or is fixed to the rear wall of the vagina in the hysterectomized patients. Similarly to what has been disclosed with reference to the first portion 2a, also the length of the second portion 2b can be adjusted, i.e. reduced, by the surgeon on the basis of the length of the rear vaginal wall to be reinforced and/or if there is a weakening of the rectovaginal fascia that can be the cause of enterocele or rectocele.

The first side arm 3a and the second side arm 3b of the first pair of arms 3 (that defines the height of the surgical suspension of the pelvic organs) are caused to pass in laparoscopy under the parietal peritoneum and are fixed to the inner fascia of the transverse muscle of the abdomen through suture points in reabsorbable plurifilament, in a point that is located 3-4 cm to the side of and 8-10 cm above the upper anterior iliac spine. The first pair of arms 3 remedies the deficit originated by the collapse of the cardinal and uterosacral ligaments, bringing the anterior and posterior vaginal fornix (or the vaginal dome in the hysterectomized patients) back to the original position thereof. In this manner it is possible to reconstitute the so-called (suspension) level I of De Lancey and remove hysterocele and cystocele, as well as decrease the size of the rectocele, that (if necessary) is then resolved definitively by carrying out a posterior colpoplasty.

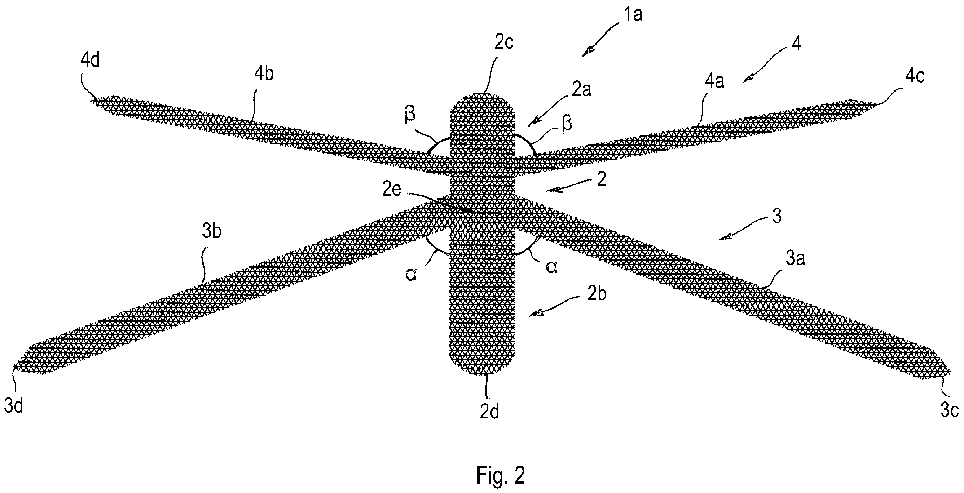

Figure 2 shows an embodiment of the mesh surgical device for laparoscopic use (mesh device) that does not form part of the invention, indicated by the number "1a". In the following, the elements of the mesh device 1a that are analogous to corresponding elements of the mesh device 1 of Figure 1 will be indicated by the same numbers and will not be disclosed in detail.

The embodiment of the mesh device of Figure 2 differs from the embodiment of the mesh device of Figure 1 inasmuch as it comprises two pairs of appendages or side arms.

The mesh device 1a comprises the (previously described) central body 2, the first pair of (previously described) side arms 3 and a second pair of side arms 4. The second pair of side arms 4 comprises a first side arm 4a and a second side arm 4b, each of which is quadrilateral-shaped, particularly extended in length and substantially ribbon-shaped. Each side arm 4a, 4b has a transverse dimension or width of 10 mm e a longitudinal dimension or length di 200 mm (20 cm). In embodiments that are not shown, the width and/or the length can be greater or less than those disclosed above and, for example, the two side arms of the second pair can have different widths and/or lengths.

Each side arm 4a, 4b comprises a free end 4c, 4d with an acute angle and an opposite end that is joined to a corresponding side of the first portion 2a. Therefore, each side arm 4a, 4b leads from opposite sides of the first portion 2a and, consequently, from opposite sides of the central body 2. In embodiments that are not shown, each side arm has a free end that is not with an acute angle but can be, for example, rectilinear, convex or concave.

Each side arm 4a, 4b leads obliquely from the corresponding side of the first portion 2a, in such a manner that a second angle β less than 90°, and in particular a second angle β of about 80°, is defined between each side arm 4a, 4b and the first portion 2a. Consequently, as it is visible in Figure 2, each side arm 4a, 4b leads obliquely from the corresponding side of the first portion 2a in such a manner that the corresponding free end 4c, 4d is positioned at about the level of the first free end 2c of the central body 2. In embodiments that are not shown, the second angle defined between each side arm and the central body is less than 90° and greater or less than 80°. In other embodiments that are not shown, the second angle can be 90°.

Moreover, as it is visible in Figure 2, each side arm 4a, 4b leads obliquely from a zone of the first portion 2a that is substantially spaced away from the connecting portion 2e. In other words, in the mesh device 1a the second pair of side arms 4 is not adjacent to the connecting portion 2e. Moreover, in the mesh device 1a the first pair of side arms 3 and the second pair of side arms 4 face respectively the first free end 2c and the second free end 2d. In other words, the first pair of side arms 3 and the second pair of side arms 4 face opposite ends of the central body 2.

From the previous description, it can be inferred that the mesh device 1a corresponds to the mesh device 1 further provided with the second pair of side arms 4. The mesh device 1a is useful above all in case of serious prolapse, i.e. a 2^{nd} - 3^{rd} degree prolapse with a major lateral defect. This embodiment of the mesh device enables important technical effects to be obtained, i.e.: correcting the lateral defect (often associated with the central defect), which is originated from an intrinsic laxity of the pubocervical fascia (associated with, and deriving from, loss of connection of the vaginal wall with the tendinous arch of the pelvic fascia); reinforcing the upper half of the vagina by providing a more valid support to the median portion of the urethra; bringing the vagina back to the so-called level II of De Lancey; stabilizing and reinforcing the prosthesis (namely, the mesh device 1a); dividing the traction force exerted on the abdominal wall by subdividing the aforesaid force between four points rather than two.

Each side arm 4a, 4b is caused to pass in laparoscopy under the parietal peritoneum and fixed to the inner fascia of the transverse muscle of the abdomen through suture points in reabsorbable plurifilament, near a point corresponding to the upper anterior iliac spine.

The first side arm 3a and the second side arm 3b of the first pair of arms 3 (that defines the height of the surgical suspension of the pelvic organs) are caused to pass under the parietal peritoneum in laparoscopy and are fixed to the inner fascia of the transverse muscle of the abdomen through suture points in reabsorbable plurifilament, in a point that is located 3-4 cm to the side of and 8-10 cm above the upper anterior iliac spine.

The central body 2 of the mesh device 1a is used in the same manner disclosed previously with reference to the central body 2 of the mesh device 1.

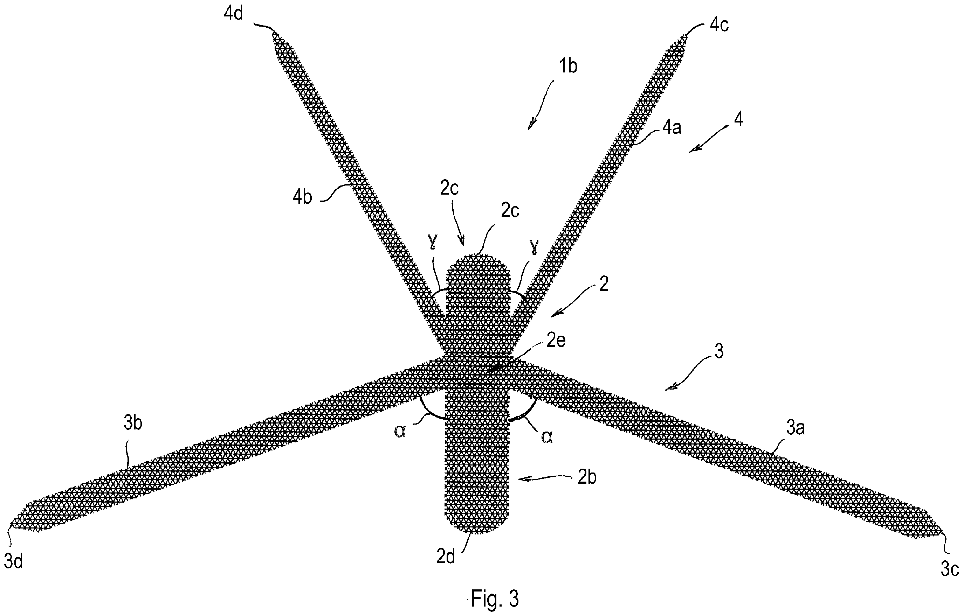

Figure 3 shows an embodiment of the mesh surgical device for laparoscopic use (mesh device) that does not form part of the invention, indicated by the number "1b". In the following, the elements of the mesh device 1b that are analogous to corresponding elements of the mesh device 1 (Figure 1) or of the mesh device 1a (Figure 2) will be indicated by the same numbers and will not be disclosed in detail.

The embodiment of the mesh device of Figure 3 is similar to the embodiment of the mesh device of Figure 2, but differs from the latter by a different size of the angles that, in the second pair of side arms 4, each side arm 4a, 4b defines with respect to the central body 2.

The mesh device 1b comprises the (previously disclosed) central body 2, the (previously disclosed) first pair of side arms 3 and the second pair of side arms 4. The second pair of side arms 4 comprises the first side arm 4a and the second side arm 4b, each of which has the shape and the dimensions disclosed previously with reference to Figure 2 and to the mesh device la.

Each side arm 4a, 4b leads obliquely from the corresponding side of the first portion 2a, in such a manner that a third angle γ less than 90°, and in particular a third angle γ of about 30°, is defined between each side arm 4a, 4b and the first portion 2a. Consequently, as it is visible in Figure 3, each side arm 4a, 4b leads obliquely from the corresponding side of the first portion 2a in such a manner that the corresponding free end 4c, 4d is not positioned at the level of the first free end 2c (as in the mesh device 1a of Figure 2), but is placed at a distance from the latter. In embodiments that are not shown, the second angle defined between each side arm and the central body is less than 90° and greater or less than 30°. As it is visible in Figure 3, each side arm 4a, 4b leads obliquely from a zone of the first portion 2a that is substantially adjacent to the connecting portion 2e. In other words, in the mesh device 1b the second pair of side arms 4 is adjacent to the connecting portion 2e. Moreover, in the mesh device 1b the first pair of side arms 3 and the second pair of side arms 4 face respectively the first free end 2c and the second free end 2d. In other words, the first pair of side arms 3 and the second pair of side arms 4 face opposite ends of the central body 2.

The mesh device 1b is useful above all in patients having a major anterior defect, for example a 2^{nd} - 3^{rd} degree hysterocele and/or cystocele, in particular associated with stress and/or urgency urinary incontinence. The second pair of side arms 4 enables important technical effects to be obtained, namely: correcting the anterior defect (deficit of the pubocervical fascia and of the pubourethral and pubovascular ligaments), in particular an anterior defect associated with stress and/or urgency urinary incontinence; stabilizing and reinforcing the prosthesis (i.e., the mesh device lb); dividing the traction force exerted on the abdominal wall by subdividing the aforesaid force between four points rather than two.

Each side arm 4a, 4b is caused to pass in laparoscopy under the parietal peritoneum, laterally to the vesical wall, and fixed to the inner fascia of the rectus muscle of the abdomen through suture points in reabsorbable plurifilament, in the suprapubic seat, 4 cm to the side of the median line (to prevent the inferior epigastric artery being damaged, which passes more laterally).

The first side arm 3a and the second side arm 3b of the first pair of arms 3 (that defines the height of the surgical suspension of the pelvic organs) are caused to pass in laparoscopy under the parietal peritoneum and are fixed to the inner fascia of the transverse muscle of the abdomen through suture points in reabsorbable plurifilament, in a point that is located 3-4 cm to the side of and 8-10 cm above the upper anterior iliac spine.

The central body 2 of the mesh device 1b is used in the same manner disclosed previously with reference to the central body 2 of the mesh device 1.

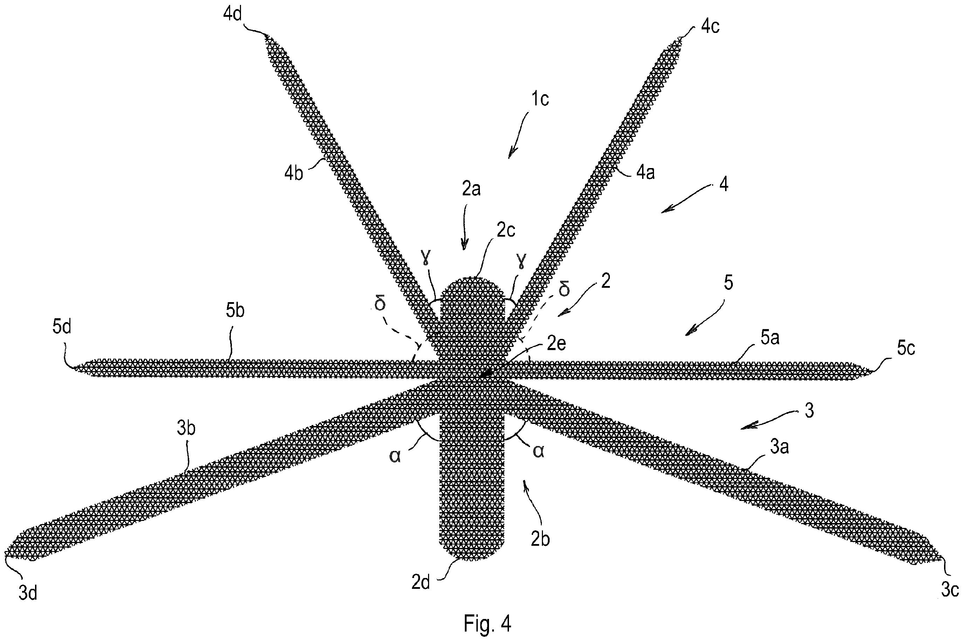

Figure 4 shows an embodiment of the mesh surgical device for laparoscopic use (mesh device) according to the invention, indicated by the number "1c". In the following, the elements of the mesh device 1c that are analogous to corresponding elements of the mesh device 1 (Figure 1), of the mesh device 1a (Figure 2) or of the mesh device 1b (Figure 3), will be indicated by the same numbers and will not be disclosed in detail.

The embodiment of the mesh device according to the invention of Figure 4 is similar to the embodiment of the mesh device of Figure 3, but differs from the latter in the presence of a third pair of side arms 5.

The mesh device 1c thus comprises the (previously disclosed) central body 2, the (previously disclosed) first pair of side arms 3, the (previously disclosed) second pair of side arms 4 and a third pair of side arms 5. It should be noted that the configuration of the second pair of side arms 4 of the mesh device 1c is the same as the configuration of the second pair of side arms 4 of the mesh device 1b. Therefore, in the mesh device 1c, the angle defined by each side arm 4a, 4b with the central body 2 is the third angle γ.

The third pair of side arms 5 comprises a first side arm 5a and a second side arm 5b, each of which is quadrilateral-shaped, particularly extended in length and substantially ribbon-shaped. Each side arm 5a, 5b has a transverse dimension or width of 10 mm and a longitudinal dimension or length of 200 mm (20 cm). In embodiments that are not shown, the width and/or the length can be greater or less than those disclosed above and, for example, the two side arms of the third pair can have widths and/or lengths that are different from one another.

Each side arm 5a, 5b comprises a free end 5c, 5d with an acute angle and an opposite end that is joined to a corresponding side of the first portion 2a. Therefore, each side arm 5a, 5b leads from opposite sides of the first portion 2a and, consequently, from opposite sides of the central body 2. In embodiments that are not shown, each side arm has a free end that is not with an acute angle but can be, for example, rectilinear or concave.

Each side arm 5a, 5b leads transversely from the corresponding side of the first portion 2a, in such a manner that a fourth angle δ of 90° (indicated by a dashed line in Figure 4) is defined between each side arm 5a, 5b and the first portion 2a. In embodiments that are not shown, the fourth angle defined between each side arm and the central body is less or greater than 90°. As it is visible in Figure 4, each side arm 5a, 5b leads from a zone of the first portion 2a that is adjacent to the connecting portion 2e. Moreover, as it is visible in Figure 4, in the mesh device 1c the third pair of side arms 5 is interposed between the first pair of side arms 3 and the second pair of side arms 4.

The mesh device 1c can be used in overweight patients of female sex, who have a major 3^{rd} - 4^{th} degree utero-vaginal prolapse, in particular a utero-vaginal prolapse associated with stress and/or urgency urinary incontinence.

The three pairs of side arms (first pair of side arms 3, second pair of side arms 4 and third pair of side arms 5) enable important technical effects to be obtained, i.e.: simultaneously correcting the anterior, lateral and central defect, when these three defects are present simultaneously; stabilizing and reinforcing further the prosthesis (i.e., the mesh device lc); dividing the traction force exerted on the abdominal wall by subdividing the aforesaid force into six points rather than two or four.

The side arms 4a, 4b of the second pair of side arms 4 are caused to pass in laparoscopy under the parietal peritoneum and fixed to the inner fascia of the rectus muscle of the abdomen through suture points in reabsorbable plurifilament, in the suprapubic seat, 4 cm to the side of the median line (to avoid damaging the lower epigastric artery, which passes more laterally).

The side arms 5a, 5b of the third pair of side arms 5 are caused to pass in laparoscopy under the parietal peritoneum and fixed to the inner fascia of the transverse muscle of the abdomen through suture points in reabsorbable plurifilament, near the point corresponding to the upper anterior iliac spine.

The first side arm 3a and the second side arm 3b of the first pair of arms 3 (that defines the height of the surgical suspension of the pelvic organs) are caused to pass in laparoscopy under the parietal peritoneum and are fixed to the inner fascia of the transverse muscle of the abdomen by suture points in absorbable plurifilament, in a point that is located 3-4 cm to the side of and 8-10 cm above the upper anterior iliac spine.

The central body 2 of the mesh device 1c is used in the same manner disclosed previously with reference to the central body 2 of the mesh device 1.

An important feature of the embodiment of mesh device 1c is that the three pairs of side arms (first pair of side arms 3, second pair of side arms 4 and third pair of side arms 5) lead from adjacent zones of the opposite sides of the central body 2. Therefore, as it is visible in Figure 4, the first side arm 3a of the first pair of side arms 3, the first side arm 4a of the second pair of side arms 4 and the first side arm 5a of the third pair of side arms 5 lead from reciprocally adjacent zones of the central body 2. Similarly, the second side arm 3b of the first pair of side arms 3, the second side arm 4b of the second pair of side arms 4 and the second side arm 5b of the third pair of side arms 5 lead from reciprocally adjacent zones of the central body 2.

The aforesaid configuration of the device 1c, namely the fact that the side arms are positioned in a reciprocally contiguous manner at each side of the central body, enables the device to be (laparoscopically) fixed onto the proximal portion of the vagina only, rather than on the entire vaginal wall, as provided for in the transvaginal prosthetic surgery. In this manner, the sub-urethral traumatism is avoided that is the cause of the most frequent postoperative complications.

As previously disclosed, in order to make the described mesh surgical device for laparoscopic use (namely the mesh device 1, 1a, 1b or 1c) it is possible to use a monofilament polypropylene non-reabsorbable mesh. One embodiment of mesh that is usable for the invention has the following features: thickness of 0.58 mm; weight of 56 g/m²; average porosity of 1.29 mm and 89.3%; diameter of the monofilament of 0.15 mm. The aforesaid mesh can also be titanized, i.e. coated with a thin layer of titanium.

Other useful properties, which the aforesaid monofilament polypropylene non-reabsorbable mesh can have, are the following: ability to form a permanent "neo-ligament" that is able to support the ligaments and fascias; ability to make a weakened vagina, to which it is applied, more tonic and resistant; inertia against infections, owing to the porosity that enables neutrophil granulocytes to pass through the warp of the mesh; stimulation of the fibroblastic reaction; permeability, to permit suitable colonization by the connective tissue; maintenance of the initial shape, without deformations; optimum biocompatibility, with consequent fewer complications (postoperative pain and inflammation).

Possible variations on and/or additions to what was disclosed above are possible.

## Claims

1. Mesh surgical device (1c) for the laparoscopic correction of a pelvic organ prolapse, comprising a central body (2) and six side arms (3a, 3b; 4a, 4b; 5a, 5b) that are configured in pairs (3, 4, 5), wherein said pairs (3, 4, 5) comprise a first pair of side arms (3) consisting of a first side arm (3a) and a second side arm (3b), said first side arm (3a) and said second side arm (3b) leading from opposite sides of said central body (2), a second pair of side arms (4) consisting of a first side arm (4a) and a second side arm (4b), said first side arm (4a) and said second side arm (4b) leading from opposite sides of said central body (2) and a third pair of side arms (5) consisting of a first side arm (5a) and a second side arm (5b), said first side arm (5a) and said second side arm (5b) leading from opposite sides of said central body (2),
**wherein** said first side arm (3a) of said first pair of side arms (3), said first side arm (4a) of said second pair of side arms (4) and said first side arm (5a) of said third pair of side arms (5) lead from reciprocally adjacent zones of said central body (2) and said second side arm (3b) of said first pair of side arms (3), said second side arm (4b) of said second pair of side arms (4) and said second side arm (5b) of said third pair of side arms (5) lead from reciprocally adjacent zones of said central body (2), such that said first side arms (3a, 4a, 5a) and said second side arms (3b, 4b, 5b) of said first, second and third pair of side arms (3, 4, 5) are positioned in a reciprocally contiguous manner at each side of said central body (2), and wherein:
said central body (2) comprises a first portion (2a) and a second portion (2b), which are reciprocally aligned and opposite and between which a connecting portion (2e) is interposed, said first portion (2a), said second portion (2b) and said connecting portion (2e) defining altogether a longitudinal dimension (L) of said central body (2);
said side arms (3a, 3b) of said first pair of side arms (3) lead from opposite sides of said connecting portion (2e) of said central body (2);
said side arms (4a, 4b) of said second pair of side arms (4) and said side arms (5a , 5b) of
said third pair of side arms (5) lead respectively from opposite sides of said first portion (2a) of said central body (2);
said first pair of side arms (3) and said second pair of side arms (4) lead obliquely from the corresponding side of said central body (2) and face opposite ends (2d, 2c) of said central body (2);
a first angle (α) of less than 90° is defined between said first side arm (3a) of said first pair (3) and said central body (2), as well as between said second side arm (3b) of said first pair (3) and said central body (2);
and a further angle (γ) of less than 90° is defined between said first side arm (4a) of said second pair (4) and said central body (2), as well as between said second side arm (4b) of said second pair (4) and said central body (2).

2. Mesh surgical device (1c) according to claim 1, wherein said first angle (α) is of 70°.

3. Mesh surgical device (1c) according to claim 1, or 2, wherein said further angle (γ) is of 30°.

4. Mesh surgical device (1c) according to any one of claims 1 to 3, wherein said first pair of side arms (3) and said second pair of side arms (4) are pointed towards opposite ends (2c, 2d) of said central body (2).

5. Mesh surgical device (1c) according to any one of claims 1 to 4, wherein a further angle (δ) of 90° is defined between said first side arm (5a) of said third pair (5) and said central body (2), as well as between said second side arm (5b) of said third pair (5) and said central body (2).

6. Mesh surgical device (1c) according to any one of claims 1 to 5, wherein said third pair of side arms (5) is interposed between said first pair of side arms (3) and said second pair of side arms (4).

## Patentansprüche

1. Chirurgische Maschenvorrichtung (1c) zur laparaskopischen Korrektur eines Beckenorganvorfalls, mit einem zentralen Körper (2) und sechs Seitenarmen (3a, 3b; 4a, 4b; 5a, 5b), die in Paaren (3, 4, 5) angeordnet sind, wobei die Paare (3, 4, 5) ein erstes Paar von Seitenarmen (3) aufweisen, bestehend aus einem ersten Seitenarm (3a) und einem zweiten Seitenarm (3b), wobei der erste Seitenarm (3a) und der zweite Seitenarm (3b) von gegenüberliegenden Seiten des mittigen Körpers (2) ausgehen, mit einem zweiten Paar von Seitenarmen (4) bestehend aus einem ersten Seitenarm (4a) und einem zweiten Seitenarm (4b), wobei der erste Seitenarm (4a) und der zweite Seitenarm (4b) von gegenüberliegenden Seiten des mittigen Körpers (2) ausgehen, und mit einem dritten Paar von Seitenarmen (5) bestehend aus einem ersten Seitenarm (5a) und einem zweiten Seitenarm (5b), wobei der erste Seitenarm (5a) und der zweite Seitenarm (5b) von gegenüberliegenden Seiten des mittigen Körpers (2) ausgehen, wobei der erste Seitenarm (3a) des ersten Paars von Seitenarmen (3), der erste Seitenarm (4a) des zweiten Paars von Seitenarmen (4) und der erste Seitenarm (5a) des dritten Paars von Seitenarmen (5) von reziprok angrenzenden Zonen des mittigen Körpers (2) und des zweiten Seitenarms (3b) des ersten Paars von Seitenarmen (3) ausgehen, wobei der zweite Seitenarm (4b) des zweiten Paars von Seitenarmen (4) und der zweite Seitenarm (5b) des dritten Paars von Seitenarmen (5) von reziprok angrenzenden Bereichen des mittigen Körpers (2) ausgehen, derart, dass die ersten Seitenarme (3a, 4a, 5a) und die zweiten Seitenarme (3b, 4b, 5b) des ersten, zweiten und dritten Paars von Seitenarmen (3, 4, 5) in einer reziprok unmittelbar angrenzenden Weise an jeder Seite des mittigen Körpers (2) angeordnet sind, und wobei: der mittige Körper (2) einen ersten Bereich (2a) und einen zweiten Bereich (2b) aufweist, die miteinander ausgerichtet und gegenüber angeordnet sind, und zwischen denen ein Verbindungsbereich (2e) angeordnet ist, wobei der erste Bereich (2a), der zweite Bereich (2b) und der Verbindungsbereich (2e) miteinander eine Längsausdehnung (L) des mittigen Körpers (2) definieren;
wobei die Seitenarme (3a, 3b) des ersten Paars von Seitenarmen (3) von gegenüberliegenden Seiten des Verbindungsbereichs (2e) des mittigen Körpers (2) ausgehen;
wobei die Seitenarme (4a, 4b) des zweiten Paars von Seitenarmen (4) und die Seitenarme (5a, 5b) des dritten Paars von Seitenarmen (5) jeweils von gegenüberliegenden Seiten des ersten Bereichs (2a) des mittigen Körpers (2) ausgehen; wobei das erste Paar von Seitenarmen (3) und das zweite Paar von Seitenarmen (4) schräg von der entsprechenden Seite des mittigen Körpers (2) ausgehen und gegenüberliegenden Enden (2d, 2c) des mittigen Körpers (2) zugewandt sind; wobei ein erster Winkel (a) von weniger als 90° zwischen dem ersten Seitenarm (3a) des ersten Paars (3) und dem mittigen Körper (2) als auch zwischen dem zweiten Seitenarm (3b) des ersten Paars (3) und dem mittigen Körper (2) definiert ist;
und wobei ferner ein Winkel (γ) von weniger als 90° zwischen dem ersten Seitenarm (4a) des zweiten Paars (4) und dem mittigen Körper (2) definiert ist, als auch zwischen dem zweiten Seitenarm (4b) des zweiten Paars (4) und dem mittigen Körper (2).

2. Chirurgische Maschenvorrichtung (1c) nach Anspruch 1, bei dem der erste Winkel (a) 70° ist.

3. Chirurgische Maschenvorrichtung (1c) nach Anspruch 1 oder 2, bei der ferner der Winkel (γ) 30° ist.

4. Chirurgische Maschenvorrichtung nach irgendeinem der Ansprüche 1 bis 3, bei der das erste Paar von Seitenarmen (3) und das zweite Paar von Seitenarmen (4) zu gegenüberliegenden Enden (2c, 2d) des mittigen Körpers (2) gerichtet sind.

5. Chirurgische Maschenvorrichtung (1c) nach irgendeinem der Ansprüche 1 bis 4, bei der ferner ein Winkel (δ) von 90° zwischen dem ersten Seitenarm (5a) des dritten Paars (5) und dem mittigen Körper (2) definiert ist, als auch zwischen dem zweiten Seitenarm (5b) des dritten Paars (5) und dem mittigen Körper (2).

6. Chirurgische Maschenvorrichtung (1c) nach irgendeinem der Ansprüche 1 bis 5, bei der das dritte Paar von Seitenarmen (5) zwischen dem ersten Paar von Seitenarmen (3) und dem zweiten Paar von Seitenarmen (4) angeordnet ist.

## Revendications

1. Dispositif chirurgical à mailles (1c) pour la correction laparoscopique d'un prolapsus d'organe pelvien, comprenant un corps central (2) et six bras latéraux (3a, 3b ; 4a, 4b ; 5a, 5b) qui sont configurés par paires (3, 4, 5), dans lequel lesdites paires (3, 4, 5) comprennent une première paire de bras latéraux (3) consistant en un premier bras latéral (3a) et un deuxième bras latéral (3b), ledit premier bras latéral (3a) et ledit deuxième bras latéral (3b) partant de côtés opposés dudit corps central (2), une deuxième paire de bras latéraux (4) consistant en un premier bras latéral (4a) et un deuxième bras latéral (4b), ledit premier bras latéral (4a) et ledit deuxième bras latéral (4b) partant de côtés opposés dudit corps central (2), et une troisième paire de bras latéraux (5) consistant en un premier bras latéral (5a) et un deuxième bras latéral (5b), ledit premier bras latéral (5a) et ledit deuxième bras latéral (5b) partant de côtés opposés dudit corps central (2),
**dans lequel** ledit premier bras latéral (3a) de ladite première paire de bras latéraux (3), ledit premier bras latéral (4a) de ladite deuxième paire de bras latéraux (4) et ledit premier bras latéral (5a) de ladite troisième paire de bras latéraux (5) partent de zones réciproquement adjacentes dudit corps central (2) et ledit deuxième bras latéral (3b) de ladite première paire de bras latéraux (3), ledit deuxième bras latéral (4b) de ladite deuxième paire de bras latéraux (4) et ledit deuxième bras latéral (5b) de ladite troisième paire de bras latéraux (5) partent de zones réciproquement adjacentes dudit corps central (2), de sorte que lesdits premiers bras latéraux (3a, 4a, 5a) et lesdits deuxièmes bras latéraux ( (3b, 4b, 5b) desdites première, deuxième et troisième paires de bras latéraux (3, 4, 5) soient positionnés de manière réciproquement contiguë de chaque côté dudit corps central (2), et dans lequel :
ledit corps central (2) comprend une première partie (2a) et une deuxième partie (2b), qui sont réciproquement alignées et opposées et entre lesquelles est intercalée une partie de liaison (2e), ladite première partie (2a), ladite deuxième partie (2b) et ladite partie de liaison (2e) définissant ensemble une dimension longitudinale (L) dudit corps central (2) ;
lesdits bras latéraux (3a, 3b) de ladite première paire de bras latéraux (3) partent de côtés opposés de ladite partie de liaison (2e) dudit corps central (2) ;
lesdits bras latéraux (4a, 4b) de ladite deuxième paire de bras latéraux (4) et lesdits bras latéraux (5a, 5b) de ladite troisième paire de bras latéraux (5) partent respectivement de côtés opposés de ladite première partie (2a) dudit corps central (2) ;
ladite première paire de bras latéraux (3) et ladite deuxième paire de bras latéraux (4) partent obliquement du côté correspondant dudit corps central (2) et font face à des extrémités opposées (2d, 2c) dudit corps central (2) ;
un premier angle (a) de moins de 90° est défini entre ledit premier bras latéral (3a) de ladite première paire (3) et ledit corps central (2), ainsi qu'entre ledit deuxième bras latéral (3b) de ladite première paire (3) et ledit corps central (2) ;
et un autre angle (y) de moins de 90° est défini entre ledit premier bras latéral (4a) de ladite deuxième paire (4) et ledit corps central (2), ainsi qu'entre ledit deuxième bras latéral (4b) de ladite deuxième paire (4) et ledit corps central (2).

2. Dispositif chirurgical à mailles (1c) selon la revendication 1, dans lequel ledit premier angle (a) est de 70°.

3. Dispositif chirurgical à mailles (1c) selon la revendication 1, ou 2, dans lequel ledit autre angle γ est de 30°.

4. Dispositif chirurgical à mailles (1c) selon l'une quelconque des revendications 1 à 3, dans lequel ladite première paire de bras latéraux (3) et ladite deuxième paire de bras latéraux (4) sont pointées vers des extrémités opposées (2c, 2d) dudit corps central (2).

5. Dispositif chirurgical à mailles (1c) selon l'une quelconque des revendications 1 à 4, dans lequel un autre angle (δ) de 90° est défini entre ledit premier bras latéral (5a) de ladite troisième paire (5) et ledit corps central (2), ainsi qu'entre ledit deuxième bras latéral (5b) de ladite troisième paire (5) et ledit corps central (2).

6. Dispositif chirurgical à mailles (1c) selon l'une quelconque des revendications 1 à 5, dans lequel ladite troisième paire de bras latéraux (5) est intercalée entre ladite première paire de bras latéraux (3) et ladite deuxième paire de bras latéraux (4).
